# EUROPEAN PATENT APPLICATION

(11) **EP 4 454 648 A1**
(43) Date of publication of application: **30.10.2024**
(21) Application number: 22911405.3
(22) Date of filing: 23.12.2022
(51) Int. Cl.: A61K 31/4418, A61P 9/00, A61P 9/04, A61P 43/00

(54) **COMPOSITION FOR IMPROVING HEART FUNCTION**

(30) Priority: 24.12.2021 JP 2021211106
(71) Applicant: Kyoto University, Kyoto-shi, Kyoto 606-8501 (JP)
(72) Inventor: KAKIZUKA, Akira, Kyoto-shi, Kyoto 606-8501 (JP); ONO, Koh, Kyoto-shi, Kyoto 606-8501 (JP); HORIE, Takahiro, Kyoto-shi, Kyoto 606-8501 (JP); TSUJI, Shuhei, Kyoto-shi, Kyoto 606-8501 (JP); WATANABE, Shin, Kyoto-shi, Kyoto 606-8501 (JP); OTANI, Chiharu, Kyoto-shi, Kyoto 606-8501 (JP); SOWA, Naoya, Kyoto-shi, Kyoto 606-8501 (JP)
(74) Representative: Hoffmann Eitle
(86) International application number: PCT/JP2022/047636
(87) International publication number: WO 2023/120704

(57) **Abstract**

The disclosure provides a composition for improving cardiac function comprising a compound of formula (I) or an ester, oxide, prodrug, pharmaceutically acceptable salt, or solvate thereof. The disclosure also provides a composition for treating heart failure comprising a compound of formula (I) or an ester, oxide, prodrug, pharmaceutically acceptable salt, or solvate thereof.

## Description

### CROSS REFERENCE TO RELATED APPLICATION

This application claims benefit of priority to Japanese Patent Application No. 2021-211106, the entire content of which is incorporated herein by reference.

### BACKGROUND

### Technical Field:

The disclosure relates to a composition for improving cardiac function. Background Art:

Heart failure is defined as "a clinical syndrome in which symptoms such as dyspnea, fatigue, and edema are manifested and exercise tolerance is decreased as a result of disruption of the compensatory mechanisms of the cardiac pump function that is caused by cardiac dysfunction, i.e. structural and/or functional cardiac abnormalities". Morbidity and mortality of heart failure is increasing due to aging of the population.

Many cases of heart failure are associated with left ventricular dysfunction. In the guidelines from the American College of Cardiology Foundation and the Japan Circulation Society heart failure is classified according to left ventricular function. Heart failure is mainly classified into heart failure with reduced left ventricular ejection fraction (LVEF) (HFrEF) and heart failure with preserved LVEF (HFpEF). HFrEF is predominantly associated with systolic dysfunction and HFpEF is predominantly associated with diastolic dysfunction. A drug for improving cardiac systolic function, i.e., a cardiotonic, is used for therapy of HFrEF, but it can cause adverse side effects by increasing systolic function only. No drug is known to improve diastolic function and effective therapy for HFpEF has not been fully established. There is a need to develop a means to improve cardiac diastolic function.

Certain 4-amino-naphthalene-1-sulfonic acid derivatives inhibit an ATPase activity of valosin-containing protein (VCP), a major ATPase in cells, and are expected to be effective for treating and/or preventing various diseases (Patent Literatures 1 to 9). Patent Literature 7 discloses that a 4-amino-naphthalene-1-sulfonic acid derivative suppresses cardiomyocyte death in ischemia reperfusion injury models.

### Patent Literature

Patent Literature 1: WO2012/014994
Patent Literature 2: WO2012/043891
Patent Literature 3: WO2014/129495
Patent Literature 4: WO2015/129809
Patent Literature 5: WO2015/033981
Patent Literature 6: WO2019/131720
Patent Literature 7: WO2019/203176
Patent Literature 8: WO2020/027137
Patent Literature 9: WO2021/079983

### SUMMARY

An object of the disclosure is to provide a new means to improve cardiac function.

The inventors have found that compounds of formula (I) improve cardiac function in heart failure models.

Accordingly, an aspect of the disclosure provides a composition for improving cardiac function comprising a compound of formula (I): 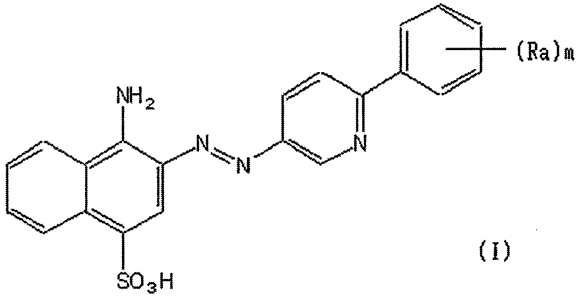 wherein
Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
m is an integer selected from 0 to 4,
or an ester, oxide, prodrug, pharmaceutically acceptable salt, or solvate thereof.

An aspect of the disclosure provides a composition for treating heart failure comprising a compound of formula (I) or an ester, oxide, prodrug, pharmaceutically acceptable salt, or solvate thereof.

The disclosure allows for improvement of cardiac function and treatment of heart failure.

### BRIEF DESCRIPTION OF DRAWINGS

Fig. 1 is a schematic diagram of the transverse aortic constriction (TAC) model.
Fig. 2 shows the experimental protocol for injecting KUS121 or 5% glucose (Tz) to mouse models of pressure overload-induced acute heart failure.
Fig. 3 shows the ratio of heart weight to body weight (left), lung weight to body weight (middle), and mRNA level of BNP (right) of sham-operated mice injected with 5% glucose, TAC mice injected with 5% glucose, and TAC mice injected with KUS121 (50 mg/kg).
Fig. 4 shows the experimental protocol for injecting KUS121 or 5% glucose to mouse models of pressure overload-induced cardiac hypertrophy.
Fig. 5 shows the heart weight (left) and ratio of heart weight to body weight (right) of sham-operated mice injected with 5% glucose, TAC mice injected with 5% glucose, and TAC mice injected with KUS121 (50 mg/kg/day).
Fig. 6 shows the quantified cardiomyocyte cross-sectional area in sham-operated mice injected with 5% glucose, TAC mice injected with 5% glucose, and TAC mice injected with KUS121 (50 mg/kg/day).
Fig. 7 shows the interventricular septum diameter at end-diastole (IVSd) (left), left ventricular ejection fraction (LVEF) (middle), and left ventricular mass (right) assessed by echocardiography in sham-operated mice injected with 5% glucose, TAC mice injected with 5% glucose, and TAC mice injected with KUS121 (50 mg/kg/day).
Fig. 8 shows the experimental protocol for injecting KUS121 or 5% glucose injection to mouse models of pressure overload-induced heart failure.
Fig. 9 shows the change in the left ventricular ejection fraction (LVEF) assessed by echocardiography in sham-operated mice injected with 5% glucose, TAC mice injected with 5% glucose, and TAC mice injected with KUS121 (50 mg/kg/day).
Fig. 10 shows the changes in the left ventricular mass (upper left), left ventricular end-diastolic diameter (LVDd) (upper right), and interventricular septum diameter at end-diastole (IVSd) (lower right) assessed by echocardiography in sham-operated mice injected with 5% glucose, TAC mice injected with 5% glucose, and TAC mice injected with KUS121 (50 mg/kg/day).
Fig. 11 shows the heart weight (upper left), ratio of heart weight to body weight (upper right), lung weight (lower left), and ratio of lung weight to body weight (lower right) of sham-operated mice injected with 5% glucose, TAC mice injected with 5% glucose, and TAC mice injected with KUS121 (50 mg/kg/day).
Fig. 12 shows the quantified cardiomyocyte cross-sectional area in sham-operated mice injected with 5% glucose, TAC mice injected with 5% glucose, and TAC mice injected with KUS121 (50 mg/kg/day).
Fig. 13 shows the quantified fibrotic area in Masson's trichrome-stained hearts of TAC mice injected with 5% glucose and TAC mice injected with KUS121 (50 mg/kg/day).
Fig. 14 shows the mRNA levels of Col1a1 and Postn in hearts of sham-operated mice injected with 5% glucose, TAC mice injected with 5% glucose, and TAC mice injected with KUS121 (50 mg/kg/day).
Fig. 15 shows the left ventricular ejection fraction (LVEF) (upper left), left ventricular end-diastolic diameter (LVDd), and heart rate (HR) assessed by echocardiography before and after KUS121 injection in TAC mice, which were subjected to TAC at 8 weeks of age and injected with KUS121 (50 mg/kg) 5 weeks later.
Fig. 16 shows the time course of the left ventricular ejection fraction from injection of KUS121 (50 mg/kg) to 150 minutes after the injection in TCA mice, which were subjected to TAC at 8 weeks of age and observed 5 weeks later by echocardiography.
Fig. 17 shows the experimental protocol for injecting KUS121 to beagle dog models of rapid ventricular pacing-induced heart failure.
Fig. 18 shows the left ventricular ejection fraction (EF: ejection fraction based on the Teichholz method and the modified-Simpson's method) and fractional shortening in left ventricle assessed by echocardiography in beagle dog models of rapid ventricular pacing-induced heart failure 15 minutes after injection of KUS (40 mg/h).
Fig. 19 shows the changes in the heart rate (upper left), systolic blood pressure (upper right), and diastolic blood pressure (lower) induced by injection of KUS121 in beagle dog models of heart failure.
Fig. 20 shows the changes in the left ventricular ejection fraction (upper left), mean pulmonary artery pressure (upper right), and pulmonary capillary wedge pressure (lower) induced by injection of KUS121 in beagle dog models of heart failure.
Fig. 21 shows the changes in the left ventricular end-diastolic pressure (left), and adjusted dp/dt (right) induced by injection of KUS121 in beagle dog models of heart failure.
Fig. 22 shows the changes in the heart rate (upper left), systolic blood pressure (upper right), cardiac output (middle left), mean pulmonary artery pressure (middle right), left ventricular end-diastolic pressure (lower left), and left ventricular dp/dt (lower right) induced by injection of dobutamine in beagle dog models of heart failure.
Fig. 23 shows a representative PV loop shift by preload reductions through an inferior vena cava occlusion at baseline (left) and during KUS121 injection (40 mg/h) (right) in beagle dog models of heart failure.
Fig.24 shows the changes from baseline in the end-systolic PV relationship (ESPVR) (left) and end-diastolic PV relationship (EDPVR) (right) obtained by PV loop shift during KUS121 injection (40 mg/h) in beagle dog models of heart failure.
Fig. 25 shows the body weight (upper left), heart weight (upper right), heart weight/tibial length (lower left), and ratio of heart weight to body weight (lower right) of control mice injected with 5% glucose, mouse models of HFpEF injected with 5% glucose, and mouse models of HFpEF injected with KUS121 (50 mg/kg).
Fig. 26 shows the change in the left ventricular ejection fraction (LVEF) in control mice injected with 5% glucose, mouse models of HFpEF injected with 5% glucose, and mouse models of HFpEF injected with KUS121 (50 mg/kg).
Fig. 27 shows the changes in the GLS (upper), E/A (middle), E/E' (lower) in control mice injected with 5% glucose, mouse models of HFpEF injected with 5% glucose, and mouse models of HFpEF injected with KUS121 (50 mg/kg).
Fig. 28 shows the changes in the blood pressure (upper) and heart rate (lower) of control mice injected with 5% glucose, mouse models of HFpEF injected with 5% glucose, and mouse models of HFpEF injected with KUS121 (50 mg/kg).
Fig. 29 shows the change in the distance traveled in treadmill test in control mice injected with 5% glucose, mouse models of HFpEF injected with 5% glucose, and mouse models of HFpEF injected with KUS121 (50 mg/kg).
Fig. 30 shows the changes in the left ventricular ejection fraction (LVEF) assessed by echocardiography before and after 5% glucose (left) or KUS187 (right) injection in TAC mice, which were subjected to TAC at 8 weeks of age and injected with 5% glucose or KUS187 (50 mg/kg) 5 weeks later.

### DETAILED DESCRIPTION

When a numerical value is accompanied with the term "about", the value is intended to represent any value in the range of -10% of the value to +10% of the value. For example, "about 20" means "a value from 18 to 22". A range defined with values of the lower and upper limits covers all values from the lower limit to the upper limit, including the values of the both limits. When a range is accompanied with the term "about", both of the limits are read as accompanied with the term. For example, "about 20 to 30" is read as "18 to 33".

Unless otherwise defined, the terms used herein are read as generally understood by those skilled in the technical fields such as organic chemistry, medical sciences, pharmaceutical sciences, molecular biology, and microbiology. Several terms used herein are defined as below. The definitions herein take precedence over the general understandings.

The term "alkyl" refers to a monovalent saturated aliphatic hydrocarbyl group having 1 to 10, preferably 1 to 6, carbon atoms. Examples of the alkyl groups include, but are not limited to, linear and branched hydrocarbyl groups, such as methyl, ethyl, n-propyl, isopropyl, n-butyl, isobutyl, sec-butyl, t-butyl, n-pentyl, and neopentyl.

The term "substituted" as a word qualifying a name of a group indicates that one or more hydrogen atoms of the group are, identically or differently, replaced with one or more designated substituents.

The term "alkylene" refers to a divalent saturated aliphatic hydrocarbyl group having 1 to 10, preferably 1 to 6, carbon atoms. The alkylene groups include branched and linear hydrocarbyl groups.

The term "alkoxy" refers to an -O-alkyl group in which the alkyl group is as defined herein. Examples of the alkoxy groups include, but are not limited to, methoxy, ethoxy, n-propoxy, isopropoxy, n-butoxy, t-butoxy, sec-butoxy, and n-pentoxy.

The term "aryl" refers to a monovalent aromatic carbocyclic group of 6 to 14 carbon atoms having a single ring (e.g., phenyl) or multiple condensed rings (e.g., naphthyl or anthryl). The aryl groups typically include phenyl and naphthyl.

The term "aryloxy" refers to an -O-aryl group in which the aryl group is as defined herein, including, e.g., phenoxy and naphthoxy.

The term "cyano" refers to the -CN group.

The term "carboxyl" or "carboxy" refers to the -COOH group or a salt thereof.

The term "carboxy ester" refers to a -C(O)O-alkyl group in which the alkyl group is as defined herein.

The term "halo" refers to a halogen, especially fluoro, chloro, bromo, or iodo.

The term "hydroxy" refers to the -OH group.

A substituent that is not explicitly defined herein is named by describing the name of the terminal functional group of the substituent first and sequentially describing the adjacent functional group(s) toward the binding point of the substituent, unless otherwise indicated. For example, the substituent "arylalkyloxycarbonyl" refers to (aryl)-(alkyl)-O-C(O)-.

Some compounds of formula (I) have enantiomers or diastereomers, depending on arrangements of their substituents. Some compounds of formula (I) may be provided as racemic mixtures or may be provided in stereoisomerically pure forms separated by a known method. Some compounds of formula (I) may be tautomers.

The term "ester" refers to an ester that may hydrolyze in vivo, including an ester which breaks down readily in a human body to leave the parent compound or a salt thereof. Suitable esters include, for example, those derived from pharmaceutically acceptable aliphatic carboxylic acids, especially alkanoic, alkenoic, cycloalkanoic, and alkanedioic acids, in which each alkyl or alkenyl group has, for example, not more than six carbon atoms. Examples of the esters include formates, acetates, propionates, butyrates, acrylates, and ethylsuccinates.

The term "oxide" refers to an oxide in which a ring-nitrogen atom in a heteroaryl group is oxidized to form N-oxide.

The term "prodrug" refers to a prodrug of a compound which may be used in contact with a human or animal tissue without an undue adverse effect such as toxicity, irritation, or allergic response within the scope of reasonable medical judgment, has a commensurate and reasonable benefit/risk ratio, and is effective in its intended use. The prodrug may be a compound which is rapidly transformed in vivo, for example by hydrolysis in blood, to yield a parent compound represented by the formula above. A general discussion is provided in T. Higuchi and V. Stella, Pro drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987, both of which are incorporated herein by reference.

The term "pharmaceutically acceptable salt" may indicate a salt of a compound of formula (I) with an inorganic or organic acid. Preferred salts include salts with inorganic acids, such as hydrochloric acid, hydrobromic acid, phosphoric acid, and sulfuric acid, and salts with organic carboxylic acids and sulfonic acids, such as acetic acid, trifluoroacetic acid, propionic acid, maleic acid, fumaric acid, malic acid, citric acid, tartaric acid, lactic acid, benzoic acid, methanesulfonic acid, ethanesulfonic acid, benzenesulfonic acid, toluenesulfonic acid, naphthalene sulfonic acid, and naphthalene disulfonic acid.

Pharmaceutically acceptable salts also include salts with conventional bases, such as alkali metal salts, e.g., a sodium salt and a potassium salt, alkaline earth metal salts, e.g., a calcium salt and a magnesium salt, ammonium salts derived from ammonia and organic amines, e.g., diethylamine, triethylamine, ethyldiisopropylamine, procaine, dibenzylamine, N-methylmorpholine, dihydroabiethylamine, methylpiperidine, L-arginine, creatine, choline, L-lysine, ethylenediamine, benzathine, ethanolamine, meglumine, and tromethamine, especially a sodium salt.

The term "solvate" means a compound of formula (I) which is coordinated with a solvent molecule to form a complex in a solid or liquid state. A suitable solvate is a hydrate.

The term "compound of formula (I)" as used herein is intended to include its esters, oxides, prodrugs, pharmaceutically acceptable salts, and solvates, as long as the context allows it.

In an embodiment, each Ra radical in formula (I) is independently selected from the group consisting of halo, hydroxy, alkyl, halo-substituted alkyl, alkoxy, and CHO.

In an embodiment, each Ra radical in formula (I) is independently selected from the group consisting of halo, hydroxy, alkyl, halo-substituted alkyl, and alkoxy.

In an embodiment, each Ra radical in formula (I) is independently selected from the group consisting of halo and alkyl.

In an embodiment, formula (I) has two Ra radicals which are halo and alkyl.

In an embodiment, each Ra radical in formula (I) is independently selected from the group consisting of alkyl, alkoxy, and CHO.

In an embodiment, formula (I) has three Ra radicals which are alkyl, alkoxy, and CHO.

In an embodiment, the compound of formula (I) is selected from the compounds listed in Table 1 below:

**[Table 1-1]**

| No. | Compound Name |
|---|---|
| 1 | 4-amino-3-(6-phenylpyridine-3-ylazo)naphthalene-1-sulfonic acid |
| 2 | 4-amino-3-(6-p-tolylpyridine-3-ylazo)naphthalene-1-sulfonic acid |
| 3 | 4-amino-3-(6-m-tolylpyridine-3-ylazo)naphthalene-1-sulfonic acid |
| 4 | 4-amino-3-(6-o-tolylpyridine-3-ylazo)naphthalene-1-sulfonic acid |
| 5 | 4-amino-3-(6-biphenyl-2-ylpyridine-3-ylazo)naphthalene-1-sulfonic acid |
| 6 | 3-[6-(2-acetylphenyl)pyridine-3-ylazo]-4-aminonaphthalene-1-sulfonic acid |
| 7 | 3-[6-(3-acetylphenyl)pyridine-3-ylazo]-4-aminonaphthalene-1-sulfonic acid |
| 8 | 3-[6-(4-acetylphenyl)pyridine-3-ylazo]-4-aminonaphthalenesulfonic acid |
| 9 | 4-amino-3-[6-(2,4-dichlorophenyl)pyridine-3-ylazo]naphthalene-1-sulfonic acid |
| 10 | 4-amino-3-[6-(2-trifluoromethylphenyl)pyridine-3-ylazo]naphthalene-1-sulfonic acid |
| 11 | 4-amino-3-[6-(4-trifluoromethylphenyl)pyridine-3-ylazo]naphthalene-1-sulfonic acid |
| 12 | 4-amino-3-[6-(2-chlorophenyl)pyridine-3-ylazo]naphthalene-1-sulfonic acid |
| 13 | 4-amino-3-[6-(3-chlorophenyl)pyridine-3-ylazo]naphthalene-1-sulfonic acid |
| 14 | 4-amino-3-[6-(4-chlorophenyl)pyridine-3-ylazo]naphthalene-1-sulfonic acid |
| 15 | 4-amino-3-[6-(2-methoxyphenyl)pyridine-3-ylazo]naphthalene-1-sulfonic acid |
| 16 | 4-amino-3-[6-(4-methoxyphenyl)pyridine-3-ylazo]naphthalene-1-sulfonic acid |
| 17 | 4-amino-3-[6-(2-isopropoxyphenyl)pyridine-3-ylazo]naphthalene-1-sulfonic acid |
| 18 | 4-amino-3-[6-(4-isopropoxyphenyl)pyridine-3-ylazo]naphthalene-1-sulfonic acid |
| 19 | 4-amino-3-[6-(2-phenoxyphenyl)pyridine-3-ylazo]naphthalene-1-sulfonic acid |
| 20 | 4-amino-3-[6-(3-methoxyphenyl)pyridine-3-ylazo]naphthalene-1-sulfonic acid |

**[Table 1-2]**

| | |
|---|---|
| 21 | 4-amino-3-[6-(2,3-dimethylphenyl)pyridine-3-ylazo]naphthalene-1-sulfonic acid |
| 22 | 4-amino-3-[6-(2,5-dimethylphenyl)pyridine-3-ylazo]naphthalene-1-sulfonic acid |
| 23 | 4-amino-3-[6-(3,5-dimethylphenyl)pyridine-3-ylazo]naphthalene-1-sulfonic acid |
| 24 | 4-amino-3-[6-(3-trifluoromethylphenyl)pyridine-3-ylazo]naphthalene-1-sulfonic acid |
| 25 | 4-{4-[5-(1-amino-4-sulfonaphthalene-2-ylazo)pyridine-2-yl]phenyl}-4-oxobutyric acid |
| 26 | 4-amino-3-(6-biphenyl-3-ylpyridine-3-ylazo)naphthalene-1-sulfonic acid |
| 27 | 4-amino-3-[6-(3-cyanophenyl)pyridine-3-ylazo]naphthalene-1-sulfonic acid |
| 28 | 4-amino-3-[6-(4-cyanophenyl)pyridine-3-ylazo]naphthalene-1-sulfonic acid |
| 29 | 4-amino-3-[6-(3,5-bistrifluoromethylphenyl)pyridine-3-ylazo]naphthalenesulfonic acid |
| 30 | 4-amino-3-[6-(4-benzoylphenyl)pyridine-3-ylazo]naphthalene-1-sulfonic acid |
| 31 | 4-amino-3-[6-(2-propoxyphenyl)pyridine-3-ylazo]naphthalene-1-sulfonic acid |
| 32 | 4-amino-3-[6-(4-fluoro-2-methylphenyl)pyridine-3-ylazo]naphthalene-1-sulfonic acid |
| 33 | 4-amino-3-[6-(5-fluoro-2-propoxyphenyl)pyridine-3-ylazo]naphthalene-1-sulfonic acid |
| 34 | 4-amino-3-[6-(2-fluoro-6-propoxyphenyl)pyridine-3-ylazo]naphthalene-1- sulfonic acid |
| 35 | 4-amino-3-[6-(4-fluoro-2-propoxyphenyl)pyridine-3-ylazo]naphthalene-1- sulfonic acid |
| 36 | 4-amino-3-[6-(5-fluoro-2-methylphenyl)pyridine-3-ylazo]naphthalene-1-sulfonic acid |
| 37 | 4-amino-3-[6-(2-fluoro-5-methylphenyl)pyridine-3-ylazo]naphthalene-1-sulfonic acid |
| 38 | 4-amino-3-[6-(2-butoxyphenyl)pyridine-3-ylazo]naphthalene-1-sulfonic acid |
| 39 | 4-amino-3-[6-(2-hexyloxyphenyl)pyridine-3-ylazo]naphthalene-1-sulfonic acid |
| 40 | 4-amino-3-[6-(4-butylphenyl)pyridine-3-ylazo]naphthalene-1-sulfonic acid |

**[Table 1-3]**

| | |
|---|---|
| 41 | 4-amino-3-[6-(2-hydroxyphenyl)pyridine-3-ylazo]naphthalene-1-sulfonic acid |
| 42 | 4-amino-3-{6-[2-(6-hydroxyhexyloxy)phenyl]pyridine-3-ylazo}naphthalene-1- sulfonic acid |
| 43 | 4-{2-[5-(1-amino-4-sulfonaphthalene-2-ylazo)pyridine-2-yl]phenoxy}butyric acid |
| 44 | 4-amino-3-{6-[2-(3-hydroxypropoxy)phenyl]pyridine-3-ylazo}naphthalene-1- sulfonic acid |
| 45 | 4-amino-3-[6-(2-isobutoxyphenyl)pyridine-3-ylazo]naphthalene-1-sulfonic acid |
| 46 | 4-amino-3-[6-(5-chloro-2-hydroxyphenyl)pyridine-3-ylazo]naphthalene-1-sulfonic acid |
| 47 | 4-amino-3-[6-(4-methylbiphenyl-2-yl)pyridine-3-ylazo]naphthalene-1-sulfonic acid |
| 48 | 4-amino-3-[6-(4'-chloro-4-methylbiphenyl-2-yl)pyridine-3-ylazo]naphthalene-1-sulfonic acid |
| 49 | 4-amino-3-[6-(4,3',5'-trimethylbiphenyl-2-yl)pyridine-3-ylazo]naphthalene-1-sulfonic acid |
| 50 | 4-amino-3-[6-(3'-chloro-4-methylbiphenyl-2-yl)pyridine-3-ylazo]naphthalene-1-sulfonic acid |
| 51 | 4-amino-3-[6-(2,6-dimethylphenyl)pyridine-3-ylazo]naphthalene-1-sulfonic acid |
| 52 | 4-amino-3-[6-(3-formyl-2-isopropoxy-5-methylphenyl)pyridine-3-ylazo]naphthalene-1-sulfonic acid |
| 53 | 4-amino-3-[6-(3-formyl-2-butoxy-5-methylphenyl)pyridine-3-ylazo]naphthalene-1-sulfonic acid |

In an embodiment, 4-amino-3-[6-(4-fluoro-2-methylphenyl)pyridine-3-ylazo]naphthalene-1-sulfonic acid, which is represented by the following formula: 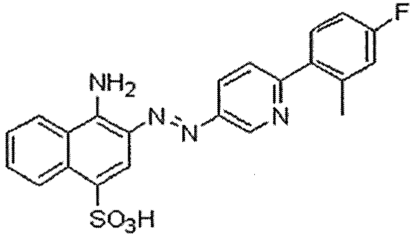 or an ester, oxide, prodrug, pharmaceutically acceptable salt or solvate thereof, preferably a sodium salt, is used.

In an embodiment, 4-amino-3-[6-(3-formyl-2-butoxy-5-methylphenyl)pyridine-3-ylazo]naphthalene-1-sulfonic acid, which is represented by the following formula: 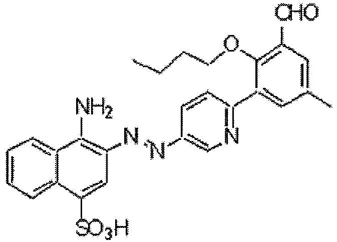 or an ester, oxide, prodrug, pharmaceutically acceptable salt or solvate thereof, preferably a sodium salt, is used.

The characteristics of the compounds of formula (I), especially the compounds listed above, and the methods for synthesizing them are described in WO2012/014994 (Patent Literature 1) in detail.

In the Examples below, a compound of formula (I) ameliorated compensatory cardiac hypertrophy and suppressed exacerbation of heart failure in mouse models of pressure overload-induced heart failure. The compound of formula (I) also lowered left ventricular end-diastolic pressure and improved systolic and diastolic function of left ventricle in beagle dog models of pacing-induced heart failure. Furthermore, the compound of formula (I) improved left ventricular diastolic function and exercise tolerance in models of heart failure with preserved LVEF (HFpEF).

Accordingly, in an aspect, a compound of formula (I) may be used for improving cardiac function. The cardiac function can be assessed by any method known in the art, and is typically assessed by non-invasive echocardiography. Left ventricular systolic function can be assessed, for example, by left ventricular ejection fraction. Left ventricular diastolic function can be assessed, for example, by patterns of blood flow velocity in mitral valve opening or mitral annulus motion velocity. Right ventricular systolic function can be assessed, for example, by tricuspid annular plane systolic excursion or right ventricular fractional area change. Right ventricular diastolic function can be assessed, for example, by patterns of right ventricular blood inflow velocity. A compound of formula (I) can improve at least one of left ventricular systolic function, left ventricular diastolic function, right ventricular systolic function, or right ventricular diastolic function. In an embodiment, a compound of formula (I) can improve cardiac function of a patient with heart failure.

In another aspect, a compound of formula (I) can be used for treating heart failure. In terms of progression of heart failure, for example, stage classification of heart failure by the American College of Cardiology Foundation/American Heart Association (ACCF/AHA) is known. In the present disclosure, the heart failure includes heart failure with symptoms of heart failure caused by structural cardiac disease (Stage C), and heart failure without symptoms of heart failure in which cardiac remodeling occurs due to compensatory mechanisms (Stage B). The heart failure in Stage C includes decompensated heart failure, in which the circulatory dynamics are not compensated, and compensated heart failure, in which the circulatory dynamics are compensated. The compensatory mechanisms include cardiac remodeling such as cardiac hypertrophy and cardiac dilatation, and increase in cardiac output caused by activation of neurohumoral factors. The heart failure may or may not be associated with cardiomyocyte death. In an embodiment, the heart failure is not associated with cardiomyocyte death.

Heart failure is classified by left ventricular systolic function into heart failure with reduced left ventricular ejection fraction (LVEF) (HFrEF), heart failure with preserved LVEF (HFpEF), and heart failure with mid-range LVEF (HFmrEF). For example, according to JCS/JHFS 2021 Guideline Focused Update on Diagnosis and Treatment of Acute and Chronic Heart Failure, the classification criteria are defined as follows; LVEF is less than 40% in HFrEF, not less than 50% in HFpEF, and not less than 40% and less than 50% in HFmrEF, though the criteria may be varied. Heart failure also can be classified by time-dependent change in LVEF into heart failure with recovered LVEF (HFrecEF), heart failure with worsened LVEF (HFworEF), and heart failure with unchanged LVEF (HFuncEF).

Conventionally, heart failure has been classified into acute heart failure, which is defined as "a pathological condition in which rapid disruption of the compensatory mechanisms of cardiac pump function causes increased ventricular end-diastolic pressure and failure of perfusion to major organs, resulting in rapid onset or worsening of the related symptoms and signs", and chronic heart failure, which is defined as "a pathological condition in which chronic failure in cardiac pump function causes persistent pulmonary and/or systemic congestion and poor tissue perfusion, resulting in problems with daily living". Acute exacerbation of chronic heart failure is included in acute heart failure.

The cause of heart failure is not limited. Examples of the causes include ischemic heart disease (such as ischemic cardiomyopathy, stunting, hibernation, and microvascular dysfunction), cardiomyopathies (such as hypertrophic cardiomyopathy, dilated cardiomyopathy, restrictive cardiomyopathy, arrhythmogenic right ventricular cardiomyopathy, noncompaction, and takotsubo cardiomyopathy; including genetic abnormalities), cardiotoxic substances (addictive substances such as alcohol, cocaine, amphetamines, and anabolic steroids, heavy metals such as copper, iron, lead, cobalt, and mercury, drugs such as anticancer drugs (e.g., anthracyclines), immunosuppressive drugs, antidepressants, antiarrhythmic drugs, NSAIDs, and anesthetics, and radiation injury), myocarditis (such as viral, bacterial, and rickettsial infections, and Chagas disease), immune disorders (such as rheumatoid arthritis, systemic lupus erythematosus, polymyositis, mixed connective tissue disease), pregnancy (such as peripartum cardiomyopathy including postpartum cardiomyopathy), infiltrative diseases (such as sarcoidosis, amyloidosis, hemochromatosis, malignant tumor invasion), endocrine diseases (such as hyperthyroidism, Cushing's disease, pheochromocytoma, adrenal insufficiency, abnormal growth hormone secretion), metabolic diseases (such as diabetes), congenital enzyme abnormalities (such as Fabry's disease, Pompe disease, Hurler syndrome, and Hunter syndrome), muscle diseases (such as muscular dystrophy and laminopathy), hypertension, valvulopathy, structural heart abnormalities (such as congenital valvular disease, atrial septal defect, ventricular septal defect, other congenital heart disease, aortic and mitral valve disease), epicardial abnormalities (such as constrictive pericarditis and cardiac tamponade), endocardial abnormalities (such as eosinophilic endocardial disease and endocardial fibroelastosis), high-output heart failure (such as severe anemia, hyperthyroidism, Paget's disease, arteriovenous shunts, pregnancy, beriberi heart), increased fluid volume (such as renal failure, excessive infusion), tachyarrhythmia (such as atrial fibrillation, atrial tachycardia, ventricular tachycardia), and bradyarrhythmia (such as sick sinus syndrome and atrioventricular block). In an embodiment, the cause of heart failure is not a heart disease with cardiomyocyte death. In an embodiment, the cause of heart failure is not myocardial infarction, hypertension, or dilated cardiomyopathy.

The term "treating heart failure" or "treatment of heart failure" as used herein means delaying or stopping the progression of heart failure and/or reducing, ameliorating, improving or eliminating heart failure in a subject suffering from heart failure.

The subject to which a compound of formula (I) may be administered include animals, typically mammals (e.g., humans, mice, rats, hamsters, rabbits, cats, dogs, cows, sheep, or monkeys), especially humans.

A compound of formula (I) may be administered in any way through a generally known administration route, for example, by oral administration, parenteral administration, injection, or infusion. Parenteral administration may be systemic administration or topical administration. Examples of the parenteral administration include intravenous, intraarterial, intradermal, subcutaneous, transdermal, intramuscular, intraperitoneal or intranasal administration. In an embodiment, a compound of formula (I) is administered intravenously to a subject. In an embodiment, a compound of formula (I) is administered orally to a subject.

A composition comprising a compound of formula (I) may be administered. The composition may be solid, liquid, or any form in between (e.g., semi-solid). The composition may be in any known dosage form which is selected according to the characteristics of the subject, the method of administration, and the dosage. Examples of the oral dosage forms include granules, fine granules, powders, coated tablets, tablets, dispersions, capsules, microcapsules, chewable tablets, liquids, suspensions, and emulsions. As an injectable dosage form, a conventional dosage form of a pharmaceutical formulation, such as a formulation for intravenous injection or infusion, or a formulation for sustained release of an active ingredient, may be used. Examples of the dosage forms for intravenous injection or infusion include an aqueous or non-aqueous solution or suspension for injection. Injectable dosage forms may be provided in sealed ampoules or vials. Injectable dosage forms may also be provided as lyophilized products that can be readily used by adding sterile liquid (e.g., water for injection) just prior to use. Injectable solutions or suspensions may be prepared from powders, granules, or tablets.

Such a dosage form can be manufactured by formulating an active ingredient by a conventional method. If necessary for the formulation, any of various pharmaceutically acceptable excipients may be added. Any excipient may be used in accordance with the employed dosage form. Examples of the excipients include buffering agents, surfactants, stabilizers, preservatives, fillers, diluents, additives, disintegrants, binders, coating agents, lubricants, lubricating agents, flavoring agents, sweeteners, solubilizers, antioxidants, bacteriostatic agents, tonicity agents, suspending agents, thickening agents, coloring agents, dyes, and flavoring agents.

Dosage and number of doses of a compound of formula (I) may be appropriately set by those skilled in the art on the basis of factors such as the animal species, health condition, age, and weight of the subject, the administration route, and the employed dosage form so that an effective amount of the compound is administered to the subject. The effective amount in a given situation may be determined by routine experimentation. For example, a compound of formula (I) may be administered in the range of about 0.001 to about 1000 mg/kg body weight/day, about 0.01 to about 200 mg/kg body weight/day, or about 0.1 to about 100 mg/kg body weight/day. Alternatively, a compound of formula (I) may be continuously infused at a rate of about 0.1 to 1000 mg/kg/hr, about 1 to 500 mg/h, or about 5 to 100 mg/h.

A compound of formula (I) may be administered in a single dose or in multiple doses, or may be chronically administered. When administered in multiple doses, the compound may be administered, for example, once to several times a day, e.g., once, twice, or three times a day, daily or every few days, e.g., every one, two, three, or seven days. The duration of administration is not limited. For example, the administration may be continued until the cardiac function is improved. The administration may be interrupted temporarily.

A compound of formula (I) may be used alone or in combination with at least one further active ingredient. When some ingredients are used in combination, a dosage form containing all the ingredients or a combination of dosage forms containing the ingredients separately may be employed. Multiple ingredients may be simultaneously administered or any ingredient may be administered at a later time point, as long as the ingredients are used for improving cardiac function or treating heart failure. Two or more further active ingredients may be used in combination. For example, a composition containing at least one further active ingredient in addition to a compound of formula (I) may be used. Examples of the active ingredients suitable for use in combination include therapeutic agents for heart failure, e.g., diuretics, mineralocorticoid receptor antagonists, cardiotonics, antiarrhythmics and beta-blockers.

In addition to administration of a compound of formula (I), non-drug therapy may be carried out. Examples of the suitable therapies include cardiac resynchronization therapy and implantable cardioverter-defibrillator (ICD) therapy.

An aspect of the disclosure provides a composition for improving cardiac function comprising a compound of formula (I).

An aspect of the disclosure provides a method for improving cardiac function comprising administering an effective amount of a compound of formula (I) to a subject.

An aspect of the disclosure provides a compound of formula (I) for use in improving cardiac function.

An aspect of the disclosure provides use of a compound of formula (I) for improving cardiac function.

An aspect of the disclosure provides use of a compound of formula (I) in manufacturing a composition for improving cardiac function.

An aspect of the disclosure provides a composition for treating heart failure comprising a compound of formula (I).

An aspect of the disclosure provides a method for treating heart failure comprising administering an effective amount of a compound of formula (I) to a subject.

An aspect of the disclosure provides a compound of formula (I) for use in treating heart failure.

An aspect of the disclosure provides use of a compound of formula (I) for treating heart failure.

An aspect of the disclosure provides use of a compound of formula (I) in manufacturing a composition for treating heart failure.

For example, the disclosure provides the following embodiments:
1. A composition for improving cardiac function, comprising a compound of formula (I): 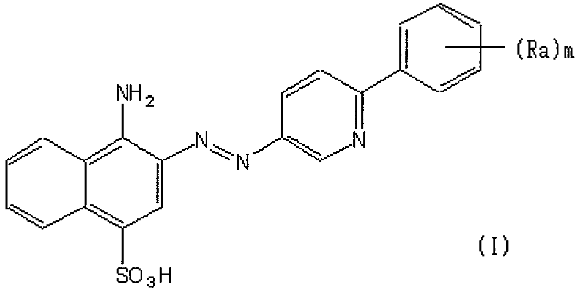 wherein
   Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
   m is an integer selected from 0 to 4,
   or an ester, oxide, prodrug, pharmaceutically acceptable salt, or solvate thereof.
2. The composition according to item 1, wherein the cardiac function is left ventricular diastolic function.
3. The composition according to item 1, wherein the cardiac function is left ventricular systolic function.
4. The composition according to item 1, wherein the cardiac function is left ventricular diastolic and systolic function.
5. The composition according to any of items 1 to 4, for improving cardiac function of a patient with heart failure.
6. A composition for treating heart failure, comprising a compound of formula (I): 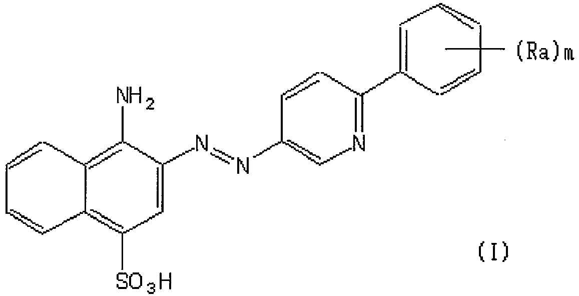 wherein
   Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
   m is an integer selected from 0 to 4,
   or an ester, oxide, prodrug, pharmaceutically acceptable salt, or solvate thereof.
7. The composition according to item 5 or 6, wherein the heart failure is heart failure with reduced left ventricular ejection fraction (LVEF) (HFrEF).
8. The composition according to item 5 or 6, wherein the heart failure is heart failure with preserved LVEF (HFpEF) or heart failure with mid-range LVEF (HFmrEF).
9. The composition according to item 8, wherein the heart failure is HFpEF.
10. The composition according to item 5 or 6, wherein the heart failure is heart failure with recovered LVEF (HFrecEF), heart failure with worsened LVEF (HFworEF), or heart failure with unchanged LVEF (HFuncEF).
11. The composition according to any one of items 5 to 10, wherein the heart failure is acute heart failure.
12. The composition according to any one of items 5 to 11, wherein the heart failure is acute exacerbation of chronic heart failure.
13. The composition according to any one of items 5 to 10, wherein the heart failure is chronic heart failure.
14. The composition according to any one of items 5 to 10, wherein the heart failure is compensated heart failure.
15. The composition according to any one of items 5 to 14, wherein the heart failure is not associated with cardiomyocyte death.
16. The composition according to any one of items 5 to 15, wherein a cause of the heart failure is ischemic heart disease, cardiomyopathy, a cardiotoxic substance, myocarditis, an immune disorder, pregnancy, an infiltrative disease, an endocrine disorder, a metabolic disease, a congenital enzyme abnormality, myopathy, hypertension, valvulopathy, an abnormal heart structure, an epicardial abnormality, an endocardial abnormality, high-output heart failure, increased fluid volume, tachyarrhythmia, or bradyarrhythmia.
17. The composition according to any one of items 5 to 16, wherein the heart failure is not caused by a heart disease associated with cardiomyocyte death.
18. The composition according to any one of items 5 to 17, wherein a cause of the heart failure is a cardiotoxic substance, myocarditis, an immune disorder, pregnancy, an infiltrative disease, an endocrine disorder, a metabolic disease, a congenital enzyme abnormality, myopathy, valvulopathy, an abnormal heart structure, an epicardial abnormality, an endocardial abnormality, high-output heart failure, increased fluid volume, tachyarrhythmia, or bradyarrhythmia.
19. The composition according to any one of items 5 to 18, wherein the heart failure is not caused by myocardial infarction, hypertension, or dilated cardiomyopathy.
20. The composition according to any one of items 1 to 19, wherein each Ra radical is independently selected from the group consisting of halo, hydroxy, alkyl, halo-substituted alkyl, alkoxy, and CHO.
21. The composition according to any one of items 1 to 20, wherein each Ra radical is independently selected from the group consisting of halo, hydroxy, alkyl, halo-substituted alkyl, and alkoxy.
22. The composition according to any one of items 1 to 21, wherein each Ra radical is independently selected from the group consisting of halo and alkyl.
23. The composition according to any one of items 1 to 22, wherein formula (I) has two Ra radicals which are halo and alkyl.
24. The composition according to any one of items 1 to 20, wherein each Ra radical is independently selected from the group consisting of alkyl, alkoxy, and CHO.
25. The composition according to any one of items 1 to 20 and 24, wherein formula (I) has three Ra radicals which are alkyl, alkoxy, and CHO.
26. The composition according to any one of items 1 to 19, wherein the compound of formula (I) is selected from the compounds listed in Table 1.
27. The composition according to any one of items 1 to 23 and 26, the compound of formula (I) is 4-amino-3-[6-(4-fluoro-2-methylphenyl)pyridine-3-ylazo]naphthalene-1-sulfonic acid.
28. The composition according to any one of items 1 to 23, 26, and 27, comprising 4-amino-3-[6-(4-fluoro-2-methylphenyl)pyridine-3-ylazo]naphthalene-1-sulfonic acid sodium salt.
29. The composition according to any one of items 1 to 21 and 24 to 26, wherein the compound of formula (I) is 4-amino-3-[6-(3-formyl-2-butoxy-5-methylphenyl)pyridine-3 -ylazo]naphthalene-1 -sulfonic acid.
30. The composition according to any one of items 1 to 21, 24 to 26, and 29, comprising 4-amino-3 - [6-(3 -formyl-2-butoxy-5 -methylphenyl)pyridine-3 - ylazo]naphthalene-1-sulfonic acid sodium salt.

The entire contents of the documents cited herein are incorporated herein by reference.

The following examples are non-limiting and provided only for describing the invention. The embodiments described above are non-limiting and may be modified without deviating from the scope of the invention as defined by the appended claims.

### EXAMPLES

### Example 1

### Materials and Methods

### Compounds

The following compounds were used.
KUS 121: 4-amino-3 - [6-(4-fluoro-2-methylphenyl)pyridine-3 -ylazo]naphthalene-1 - sulfonic acid sodium salt
KUS 187: 4-amino-3-[6-(3-formyl-2-butoxy-5-methylphenyl)pyridine-3-ylazo]naphthalene-1-sulfonic acid sodium salt
KUS 121 and KUS 187 were prepared by the method disclosed in WO2012/014994.

### Animals

Institute of Cancer Research (ICR) mice were purchased from Charles River Laboratory. Mice were maintained in temperature-controlled rooms with a 14:10 h light:dark cycle in specific pathogen-free conditions at the Institute of Laboratory Animals of Kyoto University Graduate School of Medicine. Beagle dogs were purchased from Kitayama Labes Co., Ltd. and bred at the Shiga Research Laboratory of Nissei Bailis Co. This study was approved by the Kyoto University Ethics Review Board.

### Pressure-overload cardiac hypertrophy/ heart failure model

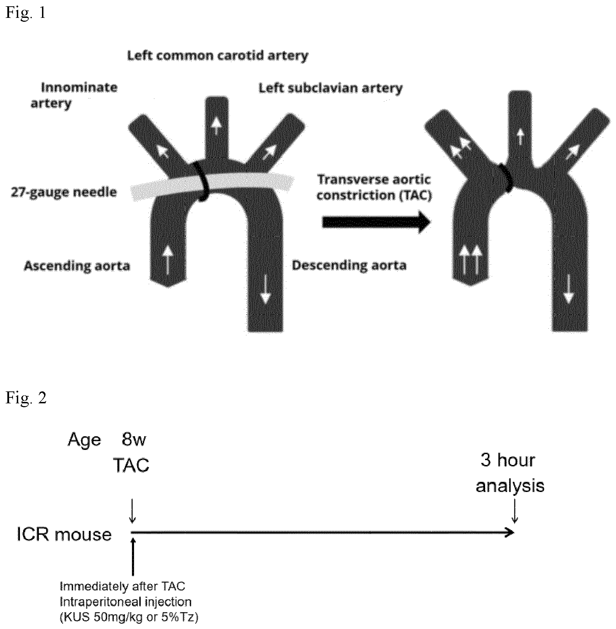

Transverse aortic constriction (TAC) was performed as described previously [Date, et al. (2002) The antioxidant N-2-mercaptopropionyl glycine attenuates left ventricular hypertrophy in in vivo murine pressure-overload model. J Am Coll Cardiol 39: 907-912.]. Briefly, 8- to 10-week-old mice were anesthetized with anesthetic (0.3 mg/kg of medetomidine, 4.0 mg/kg of midazolam, and 5.0 mg/kg of butorphanol) administered intraperitoneally, and the proximal portion of the sternum was cut open to visualize the aorta. A 7-0 silk suture was placed around the aortic arch distal to the brachiocephalic artery. The suture was tightened firmly around a blunt 26-gauge needle placed adjacent to the aorta. The needle was then removed, and the chest and overlying skin were closed. Sham-operated mice underwent an identical surgical procedure without ligation of the aortic arch. At the end of the operation, 0.3mg/kg of atipamezole was administered subcutaneously to reverse medetomidine. Fig. 1 is a schematic diagram of TAC.

### RNA extraction

To extract total RNA, tissue samples were homogenized using a polytron homogenizer in 1 mL of TriPure reagent (Sigma-Aldrich), and the total RNA was extracted in accordance with the manufacture's protocol. Total RNA extracted from cells was also isolated using 1mL of TriPure reagent. The quantity and quality of total RNA were determined using a NanoDropTM2000 spectrophotometer (Thermo Scientific).

### Quantitative real-time PCR

Total RNA was reverse transcribed by blend primer of random hexamers and oligo-dT 3:1 (volume/volume) using Verso^{™} cDNA Synthesis Kit (Thermo Fisher Scientific, AB 1453A) in accordance with the manufacturer's protocol, and the cDNA was analyzed by qRT-PCR. qRT-PCR measurements used THUNDERBIRD SYBR qPCR Mix (TOYOBO) in accordance with the manufacturer's instructions. Expression levels of messenger RNAs (mRNAs) were normalized by the indicated housekeeping genes, and calculated by the 2^{-ΔΔCt} method. All analyses were performed using a StepOnePlus real-time PCR system and StepOne Software v2.3 (Applied Biosystems, Inc).

### Wheat Germ Agglutinin (WGA) staining and the quantification

Paraffin-embedded ventricular short-axis sections were stained with FITC-conjugated lectin (Sigma, L4895). Images were captured by Axio Observer7 (Zeiss). Cardiomyocyte cross-sectional area measurement was performed using ImageJ64 software (NIH). Approximately 150-200 cells were measured per heart, and averages were used for the analysis.

### Hematoxylin-Eosin (HE) staining and the quantification

Tissues were fixed with 4% paraformaldehyde, dehydrated with 70% ethanol, and then embedded in paraffin. Deparaffinized sections were stained with hematoxylin solution for cell nucleus, and stained with eosin solution for cytoplasm. Images were captured by Axio Observer7 (Zeiss). Cardiomyocyte cross-sectional area measurement was performed using ImageJ64 software (NIH). Approximately 150-200 cells were measured per heart, and averages were used for the analysis.

### Masson trichrome staining and the quantification

As described above, deparaffinized sections were stained with hematoxylin solution for cell nucleus, Masson solution for cytoplasm, and aniline blue for collagen fiber. Images were captured by a microscopy (BZ-9000, Keyence). Aniline blue-stained regions were measured using ImageJ64 software (NIH).

### Mice Echocardiography

To analyze the cardiac function, we used Vevo^{®} 2100 (VISUALSONICS) at the indicated time points after TAC operation. Mice were kept under inhalation anesthesia with 2.5% isoflurane. LV function was measured in M mode of the parasternal short-axis view.

### Pacing-induced heart failure model in beagle dogs

After induction of general anesthesia with thiopental sodium (20 mg/kg, i.v.: 0.5 g of Labonal injection, Nipro ES Pharma Corporation) in 10- to 12-month-old male beagle dogs (9.80-11.50 kg), a tracheal cannula was inserted into the airway, and artificial respiration was performed with an Acoma veterinary ventilator (PRO-45Va, Acoma Medical Corporation). Anesthesia was maintained by inhalation of a gas mixture (Air : O2 = 3 : 0.2) and 1.0 to 2.5% isoflurane (isoflurane inhalation solution "Pfizer", Mylan Seiyaku Co., Ltd.) using an Acoma animal anesthesia machine (NS-5000A, Acoma Medical Industry Co., Ltd.) to maintain a constant depth of anesthesia. An incision was made in the right side of the neck of the animal, an animal internal cardiac pacemaker (SIP-501, Star Medical Corporation) was implanted subcutaneously, a retractable screw-in reed (Tendril STS J, St. Jude Medical, Inc.) was inserted through the right jugular vein under X-ray fluoroscopy (BV Pulsera, Koninklijke Philips N.V.), and the tip was implanted in the right ventricular wall. Pacing was started at 244-251 beats/min on postoperative day 1. Dogs with ejection fraction (EF) decreasing to 30-50% at 4 weeks after pacemaker activation were considered to have heart failure and subjected to analysis.

### Canine echocardiography

Echocardiography was performed using a general-purpose ultrasound imaging system (Vivid S6, GE Medical Systems). The left ventricular end-diastolic diameter (LVIDd), left ventricular end-systolic diameter (LVIDs), ejection fraction (EF), and left ventricular internal diameter shortening (%FS) were measured in M-mode by placing a sector probe (10 MHz) on the chest.

### Canine cardiac catheterization

Dogs were anesthetized intravenously with 14.5 to 22.3 mg/kg of secobarbital sodium (Injectable Ionar sodium (0.2), Nichi-Iko Co., Ltd.), followed by subcutaneous administration of 0.2 mg/kg of meloxicam (Metacam 0.5% injection, Boehringer Ingelheim Animal Health Japan Co.). A tracheal tube was inserted into the airway and connected to an Acoma animal ventilator (PRO-45Va, Acoma Medical Industry Co., Ltd.). The ventilator was set at a respiratory rate of 14-18 strokes/min and a tidal volume of 10-20 mL/kg/stroke, and respiration was controlled by an Acoma animal anesthesia machine (NS-5000A, Acoma Medical Industry Co., Ltd.) using a gas mixture (FiO2 = 0.3-1.0). The exhaled CO₂ partial pressure was monitored using an exhaled carbon dioxide monitor (OLG-2800, Nihon Kohden Corporation). The animals were fixed in the dorsal position and the thighs and neck were shorn. Anesthesia was maintained by continuous intravenous infusion of sodium secobarbital (5 mg/kg/hr) through the radial cutaneous vein using a Telfusion syringe pump (STC-523, Terumo Corporation). A Swan-Ganz catheter was inserted through the left femoral vein. A catheter for coronary angiography was inserted through the left femoral artery. A 5F sheath for arterial blood sampling was placed in the right femoral artery. A catheter for PV-loop analysis was inserted from the left internal carotid artery. An 8F occlusion balloon was placed from the right femoral vein for occlusion testing. In addition, KUS121 was administered from the left common jugular vein.

### Statistical analysis

Measurements are presented as means ± standard error of the mean (SEM). For other statistical comparisons, unpaired Student's t-test (two groups, parametric), Mann-Whitney test (two groups, non-parametric), or one-way analysis of variance (ANOVA) with Sidak's post-hoc test (three or more groups) were used. A p value of <0.05 was considered as statistically significant. Statistical analyses were performed using GraphPad Prism 6 (GraphPad Software, Inc.).

### Results

### Study in mouse models of heart failure during acute phase

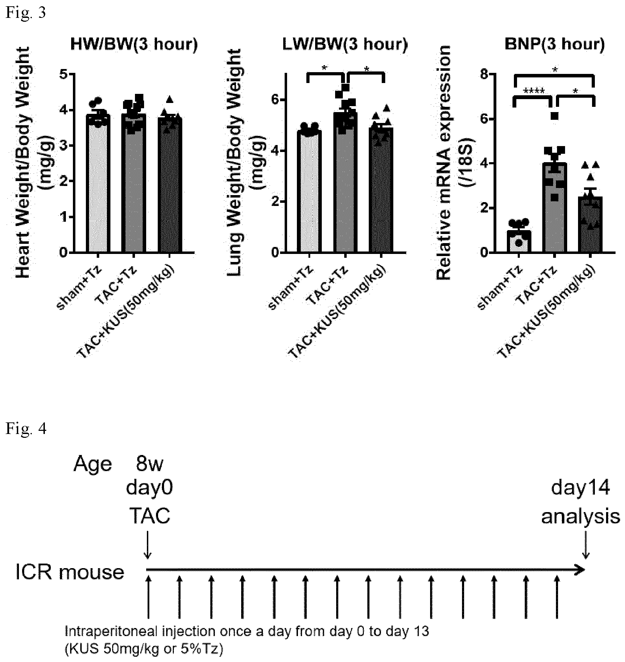

Mouse models of pressure overload-induced cardiac hypertrophy/ heart failure were prepared by transverse aortic constriction (TAC) and were injected intraperitoneally with 50 mg/kg of KUS121 immediately after TAC. Sham-operated mice and control mice were injected with 5% glucose. Three hours later, the mice were sacrificed and the body weight, heart weight, lung weight, and mRNA level of BNA were measured. The experimental protocol and the results are shown in Figs. 2 and 3, respectively. The ratio of lung weight to body weight and the mRNA level of BNP increased in the control mice, and KUS121 suppressed the increases. The ratio of heart weight to body weight was not significantly affected. These results suggest that KUS121 can attenuate left ventricular damage in the mouse models of heart failure during acute phase.

### Study in mouse models of heart failure during cardiac hypertrophic phase

Mouse models of pressure overload-induced cardiac hypertrophy/ heart failure were prepared by TAC and were injected intraperitoneally with 50 mg/kg of KUS121 daily for 2 weeks starting immediately after TAC. Sham-operated mice and control mice were injected with 5% glucose. Two weeks after TAC, echocardiography was performed, then the mice were sacrificed, the body weight and heart weight were measured, and heart samples were collected. The experimental protocol is shown in Fig. 4.

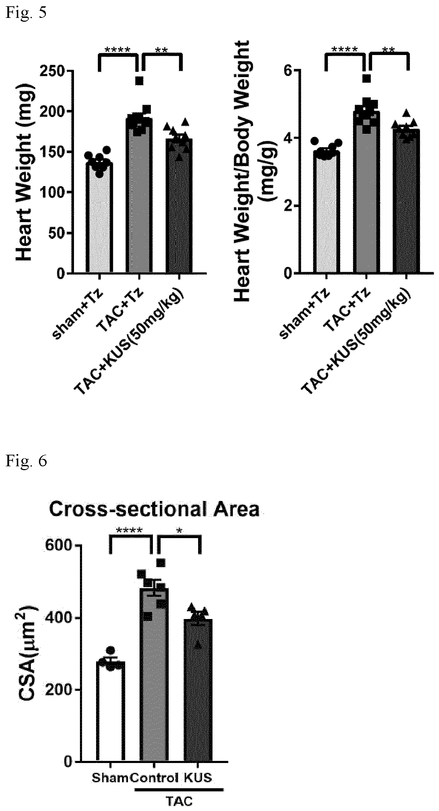

The heart weight and ratio of heart weight to body weight are shown in Fig. 5. Both increased in the control mice and KUS121 suppressed the increases. Outlines of cardiomyocytes were highlighted by WGA staining and cardiomyocyte cross-sectional area were measured. The results are shown in Fig. 6. The cardiomyocyte cross-sectional area increased in the control mice and KUS121 suppressed the increase.

In heart tissue samples mRNA levels of cardiac hypertrophy markers (BNP and ANF) and endoplasmic reticulum stress markers (Bip, CHOP, and VCP) were measured by qRT-PCR. The mRNA levels of the cardiac hypertrophy markers increased in the control mice, and KUS 121 suppressed the increases. The mRNA levels of the endoplasmic reticulum stress markers were not significantly affected.

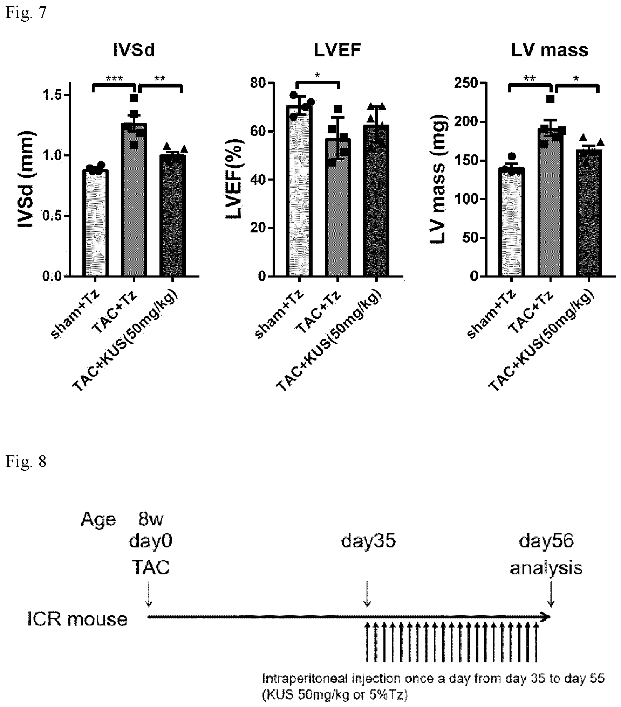

Interventricular septum diameter at end-diastole (IVSd), left ventricular mass, and left ventricular ejection fraction (LVEF) assessed by echocardiography are shown in Fig. 7. Interventricular septum and left ventricle were enlarged in the control mice and KUS121 suppressed both. Left ventricular ejection fraction was decreased in the control mice and KUS 121 did not significantly suppress the decrease.

These results suggest that KUS121 can attenuate cardiac hypertrophy in the mouse models of heart failure during cardiac hypertrophic phase.

### Study in mouse models of heart failure during heart failure phase

Mouse models of pressure overload-induced cardiac hypertrophy/ heart failure were prepared by TAC and were injected intraperitoneally with 50 mg/kg of KUS121 daily for 3 weeks starting 5 weeks after TAC. Sham-operated mice and control mice were injected with 5% glucose. Echocardiography was performed 5 weeks and 8 weeks after TAC. Eight weeks after TAC, the mice were sacrificed, the body weight and heart weight were measured, and heart samples were collected. The experimental protocol is shown in Fig. 8.

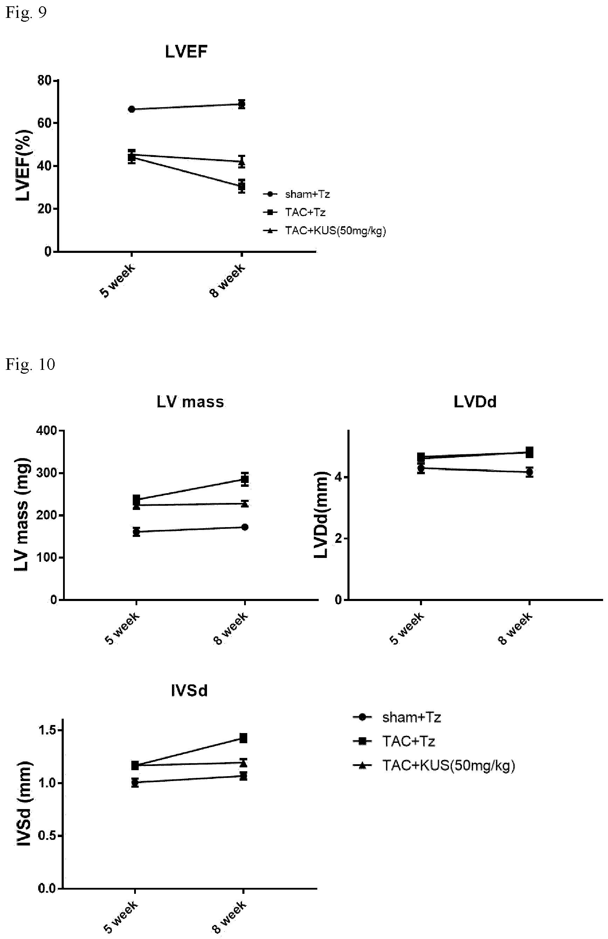

Left ventricular ejection fraction (LVEF) is shown in Fig. 9, and left ventricular mass, left ventricular end-diastolic diameter (LVDd) and interventricular septum diameter at end-diastole (IVSd) are shown in Fig. 10, all of which were assessed by echocardiography. Left ventricular systolic dysfunction was suggested in the control mice and KUS121 suppressed the dysfunction.

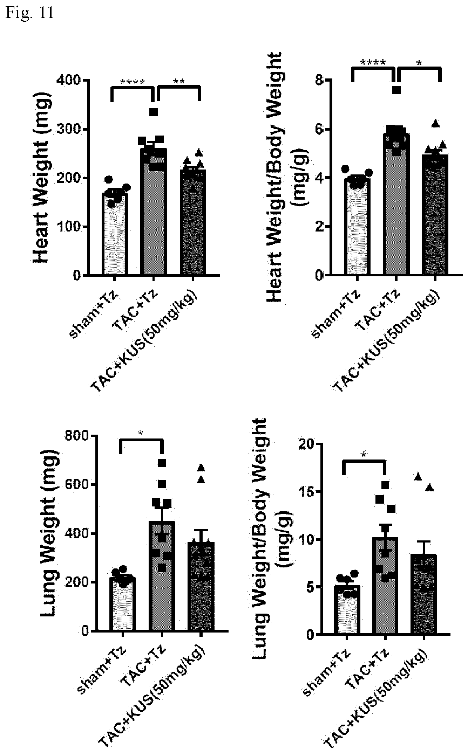

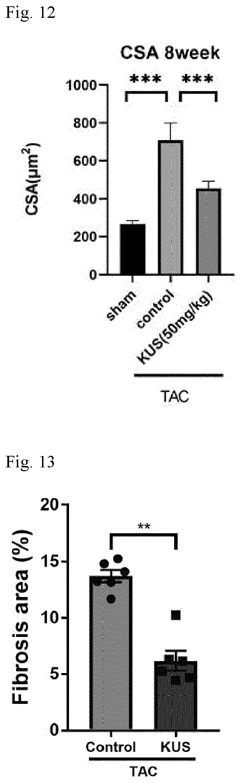

The heart weight, ratio of heart weight to body weight, lung weight, and ratio of lung weight to body weight are shown in Fig. 11. All of them were increased in the control mice, suggesting cardiac hypertrophy and pulmonary congestion. KUS121 suppressed the increase in heart weight. Cardiomyocyte cross-sectional area is shown in Fig. 12. Cardiomyocytes were enlarged in control mice and KUS121 suppressed it.

mRNA levels of cardiac hypertrophy markers (ANF and BNP) and endoplasmic reticulum stress markers (VCP, CHOP, and Bip) were measured by qRT-PCR. KUS121 increased the mRNA level of VCP, but did not significantly affect the other mRNA levels.

Fibrotic area in Masson's trichrome-stained mouse heart was quantified. The results are shown in Fig. 13. Fibrosis in the heart tissue was observed in control mice and suppressed by KUS121.

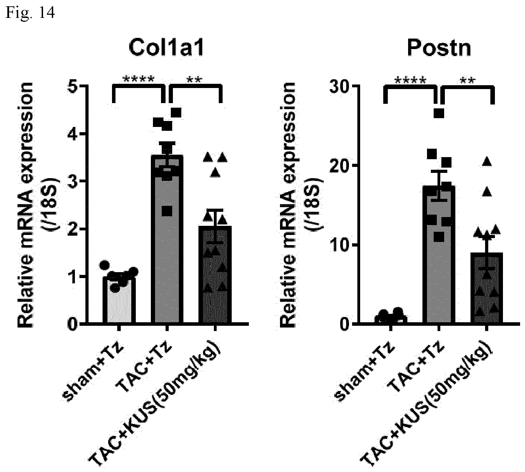

mRNA levels of cardiac fibrosis markers (Col1a1 and Postn) were measured by qRT-PCR. The results are shown in Fig. 14. The mRNA levels of Col1a1 and Postn were increased in control mice and suppressed by KUS121.

These results suggest that KUS121 can improve cardiac function and attenuate fibrosis in heart failure mice during heart failure phase.

### Acute effect of KUS121 on mouse models of heart failure

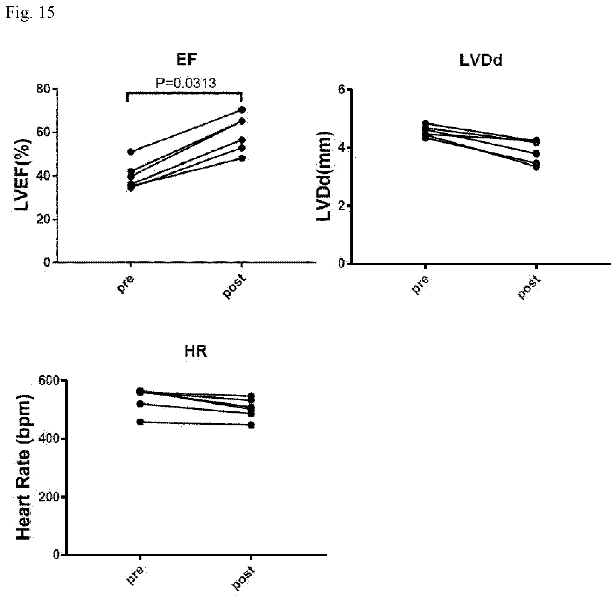

Mice were subjected to TAC at 8 weeks of age. Five weeks later (at 13 weeks of age), the mice were injected with KUS121 (50 mg/kg), and left ventricular ejection fraction (LVEF), left ventricular end-diastolic diameter (LVDd), and heart rate (HR) were assessed by echocardiography before and 10 minutes after the injection. The results are shown in Fig. 15. Recoveries in left ventricular ejection fraction and left ventricular end-diastolic diameter were observed, suggesting that KUS121 can improve cardiac function without significantly affecting heart rate.

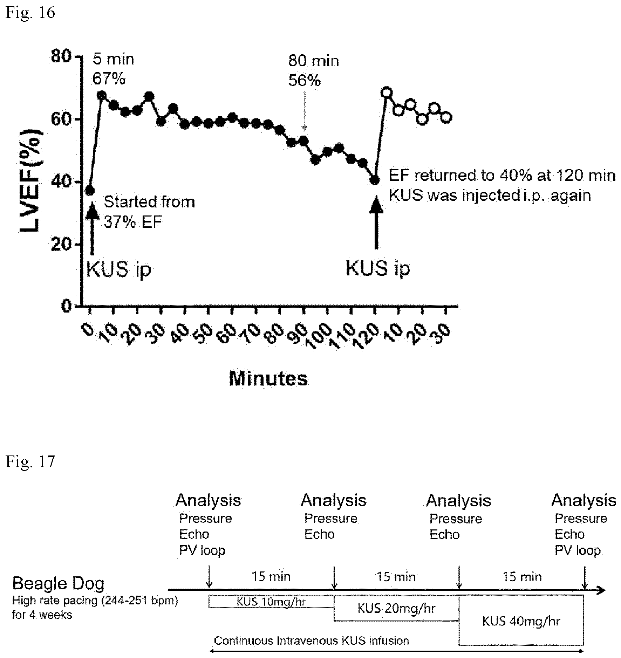

Mice were subjected to TAC at 8 weeks of age. Five weeks later (at 13 weeks of age), the mice were injected with KUS121 (50 mg/kg), and left ventricular pressure was measured by catheterization before and 10 minutes after the injection. The left ventricular pressure was 46.6 mmHg before KUS121 administration, and increased to 128.9 mmHg 10 minutes after the administration. In addition, Max/min dp/dt was changed from 1295.5/-621.4 mmHg/s to 3972.8/-1311.1 mmHg/s. Accordingly, KUS121 increased left ventricular pressure and dp/dt without affecting HR. These results suggest that KUS121 has a cardiotonic effect. Left ventricular ejection fraction was then measured over time and was 37% before KUS121 administration, increased to 67% 5 minutes after the administration, and returned to the baseline about 2 hours after the administration (Fig. 16).

### Study in heart failure model of a medium size animal

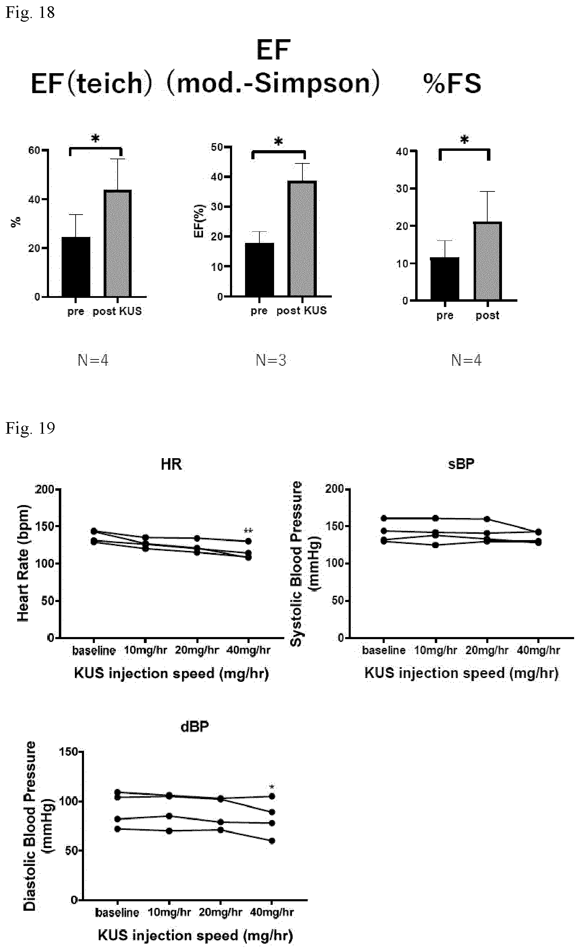

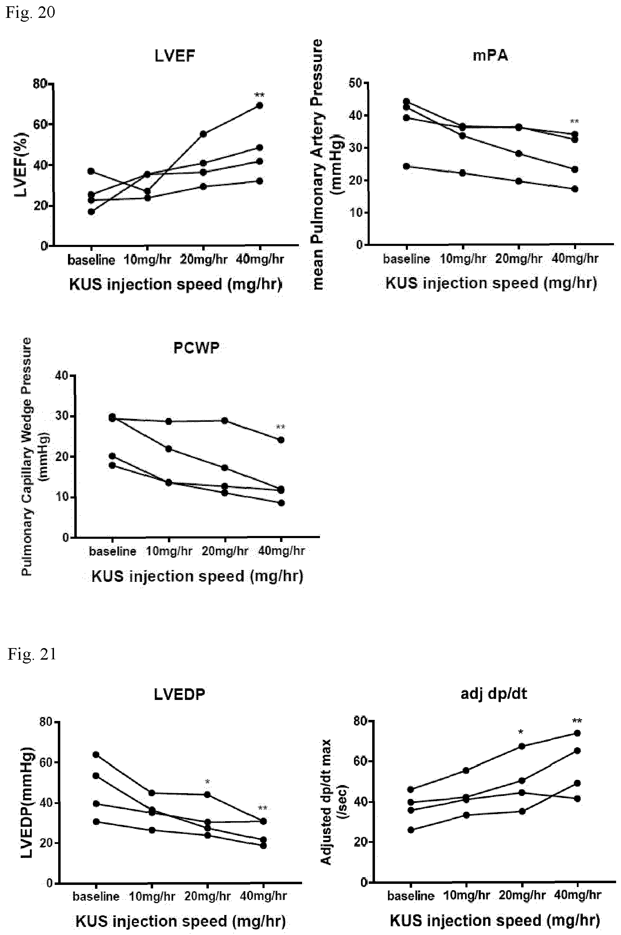

Beagle dog models of heart failure were prepared by rapid ventricular pacing. The experimental protocol of KUS121 infusion is shown in Fig. 17. Cardiac function was assessed by echocardiography before KUS121 administration and after continuous infusion at 40 mg/h. KUS121 increased LVEF, even if it was based on either of the Teichholz method and the modified-Simpson's method, and increased %FS (Fig. 18). Changes in heart rate, systolic blood pressure, diastolic blood pressure, left ventricular ejection fraction, mean pulmonary artery pressure, pulmonary capillary wedge pressure, left ventricular end-diastolic pressure, and adjusted dp/dt are shown in Figs. 19 to 21.

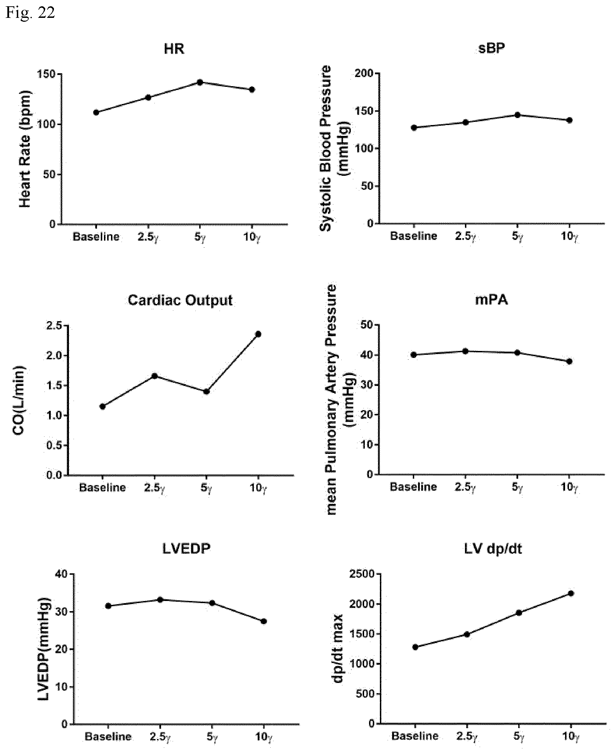

Beagle dog models of heart failure were treated according to the protocol shown in Fig. 17, using dobutamine (Fuji Pharma Co., Ltd.) at the dosage shown in Fig. 22 instead of KUS121, and cardiac function was assessed. Changes in heart rate, systolic blood pressure, cardiac output, mean pulmonary artery pressure, left ventricular end-diastolic pressure, and left ventricular dp/dt were shown in Fig. 22.

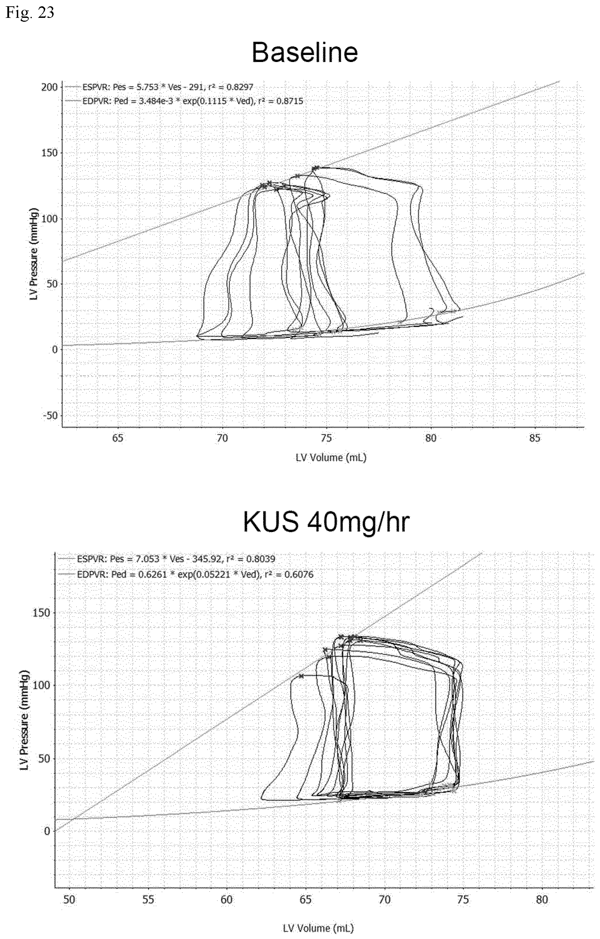

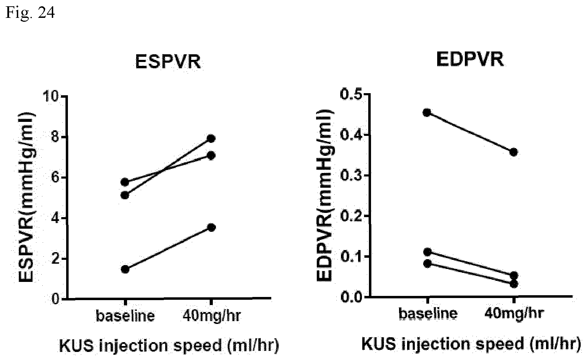

Preload reductions through an inferior vena cava occlusion was performed on beagle dog models of heart failure , and the data of pressure-volume relationship (PV loop) were collected at baseline and during KUS 121 infusion (40 mg/h). A representative PV loop shift is shown in Fig. 23. Changes from baseline in end-systolic PV relationship and end-diastolic PV relationship were obtained by the PV loop shift during KUS121 infusion and are shown in Fig. 24.

These results indicate that KUS121 lowered pulmonary artery pressure and pulmonary capillary wedge pressure, and improved left ventricular systolic and diastolic function without affecting heart rate and systolic blood pressure. According to the data from blood samples collected during acute phase, liver and kidney functions remained normal. In contrast, dobutamine, a conventionally used cardiotonic, improved left ventricular systolic function but increased heart rate.

### Example 2

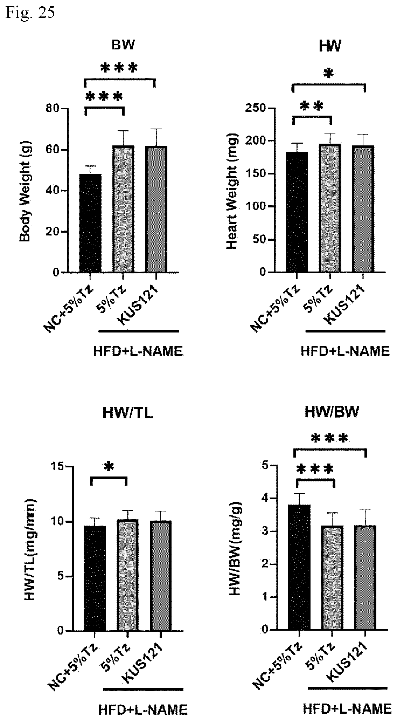

Mouse models of HFpEF were prepared by high-fat diet and administration of L-NG-nitroarginine methyl ester (L-NAME) to test the effect of KUS 121 on HFpEF. ICR mice were fed with normal diet and ultrapure water or high-fat diet and 0.5 g/L of L-NAME for 5 weeks. As expected, the high-fat diet + 0.5 g/L of L-NAME group showed a significant increase in body weight and an increase in heart weight (Fig. 25).

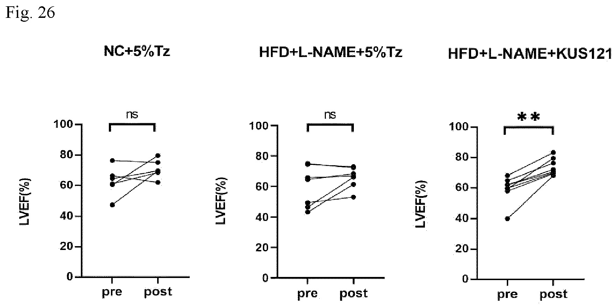

Then, acute effect of KUS121 on HFpEF was tested. ICR mice fed with normal diet or high-fat diet + 0.5 g/L of L-NAME were injected intraperitonially with 50 mg/kg of KUS121 and cardiac function was assessed by echocardiography before and 10 minutes after the injection. No difference in echocardiography was observed between the normal diet group and the high-fat diet + L-NAME group before injection of 5% glucose or KUS121 (Fig. 26). EF was not changed after 5% Tz injection in the control group and the high-fat diet + L-NAME group (Fig. 26, left and middle), but was increased after KUS121 injection in the high-fat diet + L-NAME group (Fig. 26, right). The results suggest that KUS121 can improves systolic function in HFpEF, which is associated with decrease of endogenous ATP supply as reported previously in an experiment using MRS (J Am Coll Cardiol. 2009 Jul 28;54(5):402-9).

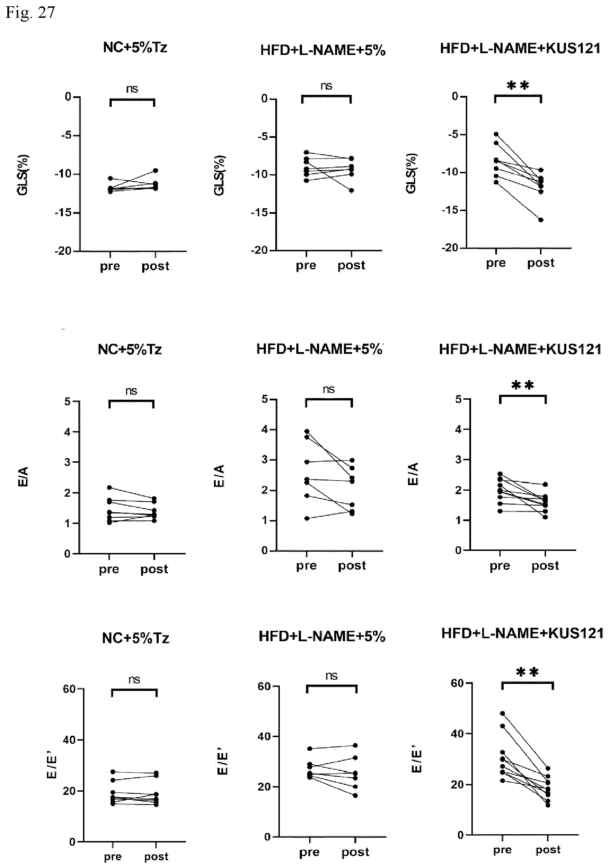

Diastolic function was also tested. Global longitudinal strain (GLS), E/A, and E/E' were assessed for evaluating cardiac diastolic function. Mice from the high-fat diet + L-NAME group had diastolic dysfunction as indicated by high levels of GLS, E/A, and E/E' before KUS121 administration, the degree of which varied among the individual animals (Fig. 27). Administration of 5% Tz did not change GLS, E/A, and E/E' in the control group and the high-fat diet L-NAME group (Fig. 27, left and middle), whereas administration of KUS121 improved GLS, E/A, and E/E' (Fig. 27, right). The results suggest an acute effect of KUS121 on diastolic dysfunction.

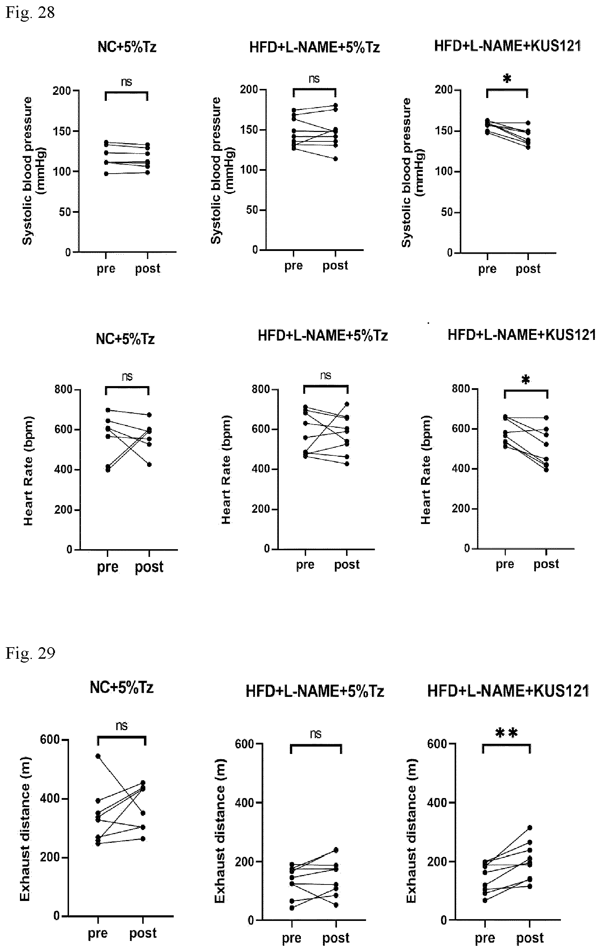

Fig. 28 shows blood pressure and heart rate before and after KUS121 administration. The high-fat diet + L-NAME group had high systolic and diastolic blood pressures (Fig. 28, upper). KUS121 administration lowered the blood pressure (Fig. 28, upper) and slightly lowered the heart rate (Fig. 28, lower).

To assess exercise tolerance, treadmill test (TMT) was performed. Mice in the high-fat diet + L-NAME group traveled shorter distance before KUS121 administration, and KUS121 improved the exercise tolerance (Fig. 29).

In conclusion, the administration of KUS121 to the model animals of HFpEF improved the diastolic function and the exercise tolerance.

### Example 3

### Acute effect of KUS187 on mouse heart failure model

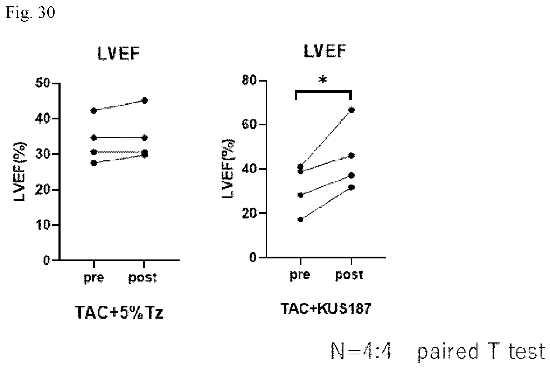

Mice were subjected to TAC at 8 weeks of age. Five weeks later (at 13 weeks of age), the mice were injected with KUS187 (50 mg/kg), and left ventricular ejection fraction was assessed by echocardiography before and 10 minutes after the injection. The results are shown in Fig. 30. The left ventricular ejection fraction was increased, indicating that KUS187 can improve cardiac function.

### Industrial Applicability

The disclosure provides the method of improving cardiac function and the method of treating heart failure and may be used in the medical field.

## Claims

1. A composition for improving cardiac function, comprising a compound of formula (I): 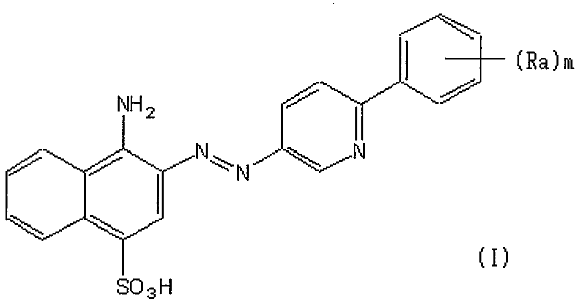 wherein
Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
m is an integer selected from 0 to 4,
or an ester, oxide, prodrug, pharmaceutically acceptable salt, or solvate thereof.

2. The composition according to claim 1, wherein the cardiac function is left ventricular diastolic function.

3. The composition according to claim 1 or 2, wherein the cardiac function is left ventricular diastolic and systolic function.

4. A composition for treating heart failure, comprising a compound of formula (I): 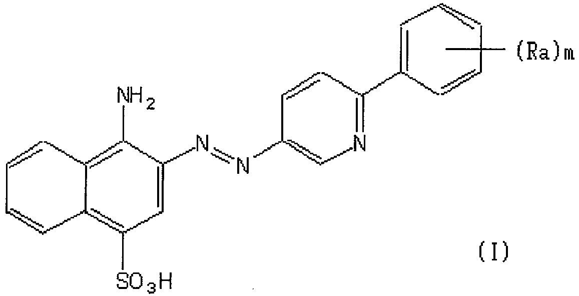 wherein
Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
m is an integer selected from 0 to 4,
or an ester, oxide, prodrug, pharmaceutically acceptable salt, or solvate thereof.

5. The composition according to claim 4, wherein the heart failure is heart failure with preserved left ventricular ejection fraction (LVEF) (HFpEF).

6. The composition according to claim 4 or 5, wherein the heart failure is acute heart failure.

7. The composition according to any one of claims 4 to 6, wherein the heart failure is not caused by a cardiac disease associated with cardiomyocyte death.

8. The composition according to any one of claims 1 to 7, wherein each Ra radical is independently selected from the group consisting of halo, hydroxy, alkyl, halo-substituted alkyl, alkoxy, and CHO.

9. The composition according to any one of claims 1 to 8, wherein each Ra radical is independently selected from the group consisting of halo, hydroxy, alkyl, halo-substituted alkyl, and alkoxy.

10. The composition according to any one of claims 1 to 7, wherein the compound of formula (I) is selected from the group consisting of
4-amino-3-(6-phenylpyridine-3-ylazo)naphthalene-1-sulfonic acid;
4-amino-3-(6-p-tolylpyridine-3-ylazo)naphthalene-1-sulfonic acid;
4-amino-3-(6-m-tolylpyridine-3-ylazo)naphthalene-1-sulfonic acid;
4-amino-3-(6-o-tolylpyridine-3-ylazo)naphthalene-1-sulfonic acid;
4-amino-3-(6-biphenyl-2-ylpyridine-3-ylazo)naphthalene-1-sulfonic acid;
3-[6-(2-acetylphenyl)pyridine-3-ylazo]-4-aminonaphthalene-1-sulfonic acid;
3-[6-(3-acetylphenyl)pyridine-3-ylazo]-4-aminonaphthalene-1-sulfonic acid;
3-[6-(4-acetylphenyl)pyridine-3-ylazo]-4-aminonaphthalenesulfonic acid;
4-amino-3-[6-(2,4-dichlorophenyl)pyridine-3-ylazo]naphthalene-1-sulfonic acid;
4-amino-3-[6-(2-trifluoromethylphenyl)pyridine-3-ylazo]naphthalene-1 - sulfonic acid;
4-amino-3-[6-(4-trifluoromethylphenyl)pyridine-3-ylazo]naphthalene-1 - sulfonic acid;
4-amino-3-[6-(2-chlorophenyl)pyridine-3-ylazo]naphthalene-1-sulfonic acid;
4-amino-3-[6-(3-chlorophenyl)pyridine-3-ylazo]naphthalene-1-sulfonic acid;
4-amino-3-[6-(4-chlorophenyl)pyridine-3-ylazo]naphthalene-1-sulfonic acid;
4-amino-3-[6-(2-methoxyphenyl)pyridine-3-ylazo]naphthalene-1-sulfonic acid;
4-amino-3-[6-(4-methoxyphenyl)pyridine-3-ylazo]naphthalene-1-sulfonic acid;
4-amino-3-[6-(2-isopropoxyphenyl)pyridine-3-ylazo]naphthalene-1-sulfonic acid;
4-amino-3-[6-(4-isopropoxyphenyl)pyridine-3-ylazo]naphthalene-1-sulfonic acid;
4-amino-3-[6-(2-phenoxyphenyl)pyridine-3-ylazo]naphthalene-1-sulfonic acid;
4-amino-3-[6-(3-methoxyphenyl)pyridine-3-ylazo]naphthalene-1-sulfonic acid;
4-amino-3-[6-(2,3-dimethylphenyl)pyridine-3-ylazo]naphthalene-1-sulfonic acid;
4-amino-3-[6-(2,5-dimethylphenyl)pyridine-3-ylazo]naphthalene-1-sulfonic acid;
4-amino-3-[6-(3,5-dimethylphenyl)pyridine-3-ylazo]naphthalene-1-sulfonic acid;
4-amino-3-[6-(3-trifluoromethylphenyl)pyridine-3-ylazo]naphthalene-1-sulfonic acid;
4-{4-[5-(1-amino-4-sulfonaphthalene-2-ylazo)pyridine-2-yl]phenyl}-4-oxobutyric acid;
4-amino-3-(6-biphenyl-3-ylpyridine-3-ylazo)naphthalene-1-sulfonic acid;
4-amino-3-[6-(3-cyanophenyl)pyridine-3-ylazo]naphthalene-1-sulfonic acid;
4-amino-3-[6-(4-cyanophenyl)pyridine-3-ylazo]naphthalene-1-sulfonic acid;
4-amino-3-[6-(3,5-bistrifluoromethylphenyl)pyridine-3-ylazo]naphthalenesulfonic acid;
4-amino-3-[6-(4-benzoylphenyl)pyridine-3-ylazo]naphthalene-1-sulfonic acid;
4-amino-3-[6-(2-propoxyphenyl)pyridine-3-ylazo]naphthalene-1-sulfonic acid;
4-amino-3-[6-(4-fluoro-2-methylphenyl)pyridine-3-ylazo]naphthalene-1-sulfonic acid;
4-amino-3-[6-(5-fluoro-2-propoxyphenyl)pyridine-3-ylazo]naphthalene-1-sulfonic acid;
4-amino-3-[6-(2-fluoro-6-propoxyphenyl)pyridine-3-ylazo]naphthalene-1 - sulfonic acid;
4-amino-3-[6-(4-fluoro-2-propoxyphenyl)pyridine-3-ylazo]naphthalene-1 - sulfonic acid;
4-amino-3-[6-(5-fluoro-2-methylphenyl)pyridine-3-ylazo]naphthalene-1-sulfonic acid;
4-amino-3-[6-(2-fluoro-5-methylphenyl)pyridine-3-ylazo]naphthalene-1-sulfonic acid;
4-amino-3-[6-(2-butoxyphenyl)pyridine-3-ylazo]naphthalene-1-sulfonic acid;
4-amino-3-[6-(2-hexyloxyphenyl)pyridine-3-ylazo]naphthalene-1-sulfonic acid;
4-amino-3-[6-(4-butylphenyl)pyridine-3-ylazo]naphthalene-1-sulfonic acid;
4-amino-3-[6-(2-hydroxyphenyl)pyridine-3-ylazo]naphthalene-1-sulfonic acid;
4-amino-3-{6-[2-(6-hydroxyhexyloxy)phenyl]pyridine-3-ylazo}naphthalene-1 - sulfonic acid;
4-{2-[5-(1-amino-4-sulfonaphthalene-2-ylazo)pyridine-2-yl]phenoxy} butyric acid;
4-amino-3-{6-[2-(3-hydroxypropoxy)phenyl]pyridine-3-ylazo}naphthalene-1 - sulfonic acid;
4-amino-3-[6-(2-isobutoxyphenyl)pyridine-3-ylazo]naphthalene-1-sulfonic acid;
4-amino-3-[6-(5-chloro-2-hydroxyphenyl)pyridine-3-ylazo]naphthalene-1-sulfonic acid;
4-amino-3-[6-(4-methylbiphenyl-2-yl)pyridine-3-ylazo]naphthalene-1-sulfonic acid;
4-amino-3-[6-(4'-chloro-4-methylbiphenyl-2-yl)pyridine-3-ylazo]naphthalene-1-sulfonic acid;
4-amino-3-[6-(4,3',5'-trimethylbiphenyl-2-yl)pyridine-3-ylazo]naphthalene-1-sulfonic acid;
4-amino-3-[6-(3'-chloro-4-methylbiphenyl-2-yl)pyridine-3-ylazo]naphthalene-1-sulfonic acid;
4-amino-3-[6-(2,6-dimethylphenyl)pyridine-3-ylazo]naphthalene-1-sulfonic acid;
4-amino-3-[6-(3-formyl-2-isopropoxy-5-methylphenyl)pyridine-3-ylazo]naphthalene-1-sulfonic acid; and
4-amino-3-[6-(3-formyl-2-butoxy-5-methylphenyl)pyridine-3-ylazo]naphthalene-1-sulfonic acid.

11. The composition according to any one of claims 1 to 10, the compound of formula (I) is 4-amino-3-[6-(4-fluoro-2-methylphenyl)pyridine-3-ylazo]naphthalene-1-sulfonic acid.

12. The composition according to any one of claims 1 to 8 and 10, wherein the compound of formula (I) is 4-amino-3-[6-(3-formyl-2-butoxy-5-methylphenyl)pyridine-3 -ylazo]naphthalene-1-sulfonic acid.
